Europäisches Patentamt

European Patent Office

Office européen des brevets

(19)

(11) Veröffentlichungsnummer: **0 017 174**
**B1**

(12) # EUROPÄISCHE PATENTSCHRIFT

(45) Veröffentlichungstag der Patentschrift:
**23.02.83**

(21) Anmeldenummer: **80101638.7**

(22) Anmeldetag: **27.03.80**

(51) Int. Cl.³: **F 25 J 3/08,** C 07 C 7/09,
C 01 B 3/50

(54) **Verfahren zum Zerlegen eines Gasgemisches.**

(30) Priorität: **30.03.79 DE 2912761**

(43) Veröffentlichungstag der Anmeldung:
**15.10.80 Patentblatt 80/21**

(45) Bekanntmachung des Hinweises auf die Patenterteilung:
**23.02.83 Patentblatt 83/8**

(84) Benannte Vertragsstaaten:
**BE DE FR GB IT**

(56) Entgegenhaltungen:
**keine**

(73) Patentinhaber: **Linde Aktiengesellschaft,**
**Abraham-Lincoln-Strasse 21, D-6200 Wiesbaden (DE)**

(72) Erfinder: **Fabian, Rainer, Alpspitzweg 5,**
**D-8192 Geretsried (DE)**

(74) Vertreter: **Schaefer, Gerhard, Dr., Linde**
**Aktiengesellschaft Zentrale Patentabteilung,**
**D-8023 Höllriegelskreuth (DE)**

ACTORUM AG

## Verfahren zum Zerlegen eines Gasgemisches

Die Erfindung betrifft ein Verfahren zum Zerlegen eines im wesentlichen Wasserstoff, Methan und Kohlenmonoxid enthaltenden Gasgemisches mit einer bei tiefer Temperatur erfolgenden Methanwäsche, die eine Waschsäule und eine Rektifiziersäule enthält, wobei das Gasgemisch zunächst bei einer über der Temperatur der Methanwäsche liegenden Temperatur einer partiellen Kondensation unterzogen und die dabei gasförmig verbleibenden Fraktionen der Methanwäsche zugeführt wird.

Bei einem bekannten Verfahren dieser Art (Hydrocarbon Processing, November 1971, Seite 164) wird ein durch eine Dampfreformierung von Kohlenwasserstoffen und nachfolgende $CO_2$-Abtrennung gewonnenes Gasgemisch auf eine Temperatur von etwa 90 K abgekühlt und einer Methanwäsche unterzogen. Dabei fällt praktisch CO-freier Wasserstoff als Kopfprodukt einer Waschsäule an, während das in Methan gelöste Kohlenmonoxid aus dem Sumpf abgezogen wird. Das beladene Waschmittel wird anschliessend in einer $CO/CH_4$-Trennsäule regeneriert und der Waschsäule erneut aufgegeben. Als Kopfprodukt der Regeneriersäule fällt dabei Kohlenmonoxid als weiteres Verfahrensprodukt an. Die für die Regenerierung des Waschmittels erforderliche Kälte wird durch einen Kreislauf bereitgestellt, in dem ein Teil des abgetrennten Kohlenmonoxids geführt wird. Dazu wird es in einem Verdichter komprimiert, kondensiert im Sumpf der Regeneriersäule unter gleichzeitiger Beheizung des Sumpfes und wird nach Entspannung der Regeneriersäule als Rücklauf aufgegeben.

Das bei diesem bekannten Verfahren im Sumpf der Regeneriersäule anfallende Methan wird, soweit es den Bedarf für die Methanwäsche übersteigt, verdampft und gemeinsam mit an anderer Stelle anfallenden Gasen als Restgas abgeführt.

Das bekannte Verfahren wird vorteilhaft zur Zerlegung eines Gasgemisches mit relativ kleinem Methangehalt, der meist unter 5% liegt, herangezogen. Dabei kann das abgetrennte Methan grösstenteils als Ersatz für Waschmittelverluste herangezogen werden. Wenn jedoch ein Gasgemisch mit höherem Methangehalt zu zerlegen ist, dann hat dies einen grossen Mehraufwand für die $CO/CH_4$-Trennung in der Regeneriersäule und damit einen stark erhöhten Aufwand für den Kohlenmonoxid-Kältekreislauf zur Folge.

Weiterhin ist aus der US-A-3 361 534 schon ein Verfahren bekannt geworden, bei dem im Rahmen der Abkühlung eines Gasstromes ein methanreiches Kondensat abgeschieden und von der Waschsäule ferngehalten wird. Die Gewinnung einer praktisch reinen Methanfraktion aus diesem Kondensat ist jedoch nur unter erheblichen Energieaufwand möglich, da reines Methan nur über den Sumpf der Regeneriersäule der Methanwäsche abgezogen wird. Da die Sumpftemperatur bei etwa 120 K liegt, bedeutet dies, dass alles zu gewinnende reine Methan bis auf diese tiefe Temperatur abgekühlt werden muss, was einen hohen Energieaufwand, der durch einen gesonderten Stickstoffkreislauf erbracht wird, erfordert.

Der Erfindung liegt deshalb die Aufgabe zugrunde, ein Verfahren der eingangs genannten Art so auszugestalten, dass die Zerlegung des Gasgemisches bei einem relativ hohen Methangehalt unter einem möglichst geringen Energieaufwand durchgeführt werden kann.

Diese Aufgabe wird dadurch gelöst, dass die bei der partiellen Kondensation kondensierte Fraktion in eine bei höherer Temperatur als die Rektifiziersäule betriebene Abtriebssäule, in der Methan als Sumpfprodukt und ein an Wasserstoff und Kohlenmonoxid angereichertes Kopfprodukt gewonnen wird, geführt wird.

Erfindungsgemäss wird damit ausgenützt, dass das im Gasgemisch enthaltene Methan bei der Abkühlung auf die für die Methanwäsche erforderliche tiefe Temperatur bereits vor Erreichen dieser Temperatur zu einem grossen Teil kondensiert. Dies erfolgt beispielsweise im Temperaturbereich zwischen etwa 140 und 180 K und ist im Einzelfall natürlich vom jeweiligen Druck des Gasgemisches abhängig. Da das kondensierte Methan entsprechend den Gleichgewichtsbedingungen nur verhältnismässig wenig Wasserstoff und Kohlenmonoxid in gelöster Form enthält, hat es sich nun als günstig erwiesen, das Kondensat bei einer mittleren Temperatur vom übrigen Gasgemisch abzuzweigen und in einer Abtriebssäule von begleitendem Wasserstoff und Kohlenmonoxid zu trennen. Der für diese Trennung erforderliche Energieaufwand ist verhältnismässig gering und wird bei weitem von den Energieeinsparungen übertroffen, die dadurch erzielt werden, dass ein Grossteil des Methans gar nicht mehr in den Tieftemperaturteil der Anlage geleitet werden muss. Der apparative Mehraufwand für die Behandlung des Kondensats wird wiederum durch Einsparungen im Tieftemperaturteil der Anlage, die wegen der kleineren Bauteile aufgrund des verringerten Gasanfalls in diesem Bereich möglich sind, weitgehend kompensiert.

Die nach dem erfindungsgemässen Verfahren bei mittlerer Temperatur abgetrennte Methanfraktion kann wieder verdampft und, weil sie praktisch kohlenmonoxidfrei ist, beispielsweise als SNG, d.h. als Erdgasersatz, in ein Versorgungsnetz eingespeist werden.

Das Austreiben des im kondensierten Methan gelösten Wasserstoffs und Kohlenmonoxids kann auf üblichem Wege erfolgen, beispielsweise durch Beheizung des Sumpfes der Abtriebssäule oder durch Einleiten eines inerten Strippgases. Als besonders günstig hat es sich erwiesen, das Kondensat vor der Einspeisung in die Abtriebssäule zu entspannen, um die Abtrennung zu erleichtern und zusätzlich im Sumpf eine Heizung vorzusehen, die beispielsweise mit Rohgas beschickt werden kann.

In einer günstigen weiteren Ausgestaltung der Erfindung wird die partielle Kondensation des Gasgemisches mehrstufig durchgeführt, um auf diese Weise einen möglichst hohen Anteil des Methans vor Eintritt in die Tieftemperatur-Methanwäsche abzutrennen. Dazu wird das bei der ersten Kondensationsstufe gasförmig verbleibende Gasgemisch nach einer weiteren Abkühlung wiederum einer Phasentrennung unterzogen, wobei das neu gebildete methanreiche Kondensat wiederum von gelöstem Wasserstoff und Kohlenmonoxid befreit wird. Diese Verfahrensweise kann mehrstufig wiederholt werden, um weiteres Methan abzutrennen. Aus wirtschaftlichen Erwägungen wird es jedoch im allgemeinen am günstigsten sein, nur zwei oder drei Kondensationsstufen vorzusehen. Bei mehr Kondensationsstufen dürfte sich der Investitionsaufwand für die zusätzlichen Kondensationsstufen stärker bemerkbar machen als die im Tieftemperaturteil ermöglichte Einsparung, zumal bei aufeinanderfolgenden Kondensationsstufen die Temperatur des Kondensats jeweils sinkt und damit ein grösserer Anteil an Wasserstoff und Kohlenmonoxid aus dem Kondensat abgetrennt werden muss. Bei Verwendung einer zweistufigen Kondensation werden dagegen die Einsparungen im Tieftemperaturteil bei weitem von den Aufwendungen für die partielle Kondensation übertroffen.

Besonders vorteilhaft ist es bei einer mehrstufigen Kondensation, wenn die Kondensate der einzelnen Stufen in eine gemeinsame Abtriebssäule eingespeist werden, um die Investitionskosten möglichst gering zu halten. Dabei erfolgt die Einspeisung der einzelnen Kondensate zweckmässig an verschiedenen Stellen, die dem Gleichgewichtsverlauf in der Abtriebssäule und den jeweiligen Kondensatzusammensetzungen angepasst sind.

Während das im Sumpf der Abtriebssäule anfallende Methan praktisch frei von Verunreinigungen ist, fällt ein Kopfprodukt an, das an Wasserstoff und Kohlenmonoxid angereichert ist, aber auch einen hohen Methananteil aufweist. Um auch aus diesem Gas wertvolle Verfahrensprodukte zu gewinnen, wird erfindungsgemäss weiter vorgeschlagen, dieses Gasgemisch auf die tiefe Temperatur der Methanwäsche abzukühlen. Dabei kondensiert nämlich der grösste Teil des Methans sowie ein Teil des Kohlenmonoxids aus, während der Wasserstoff in der Gasphase verbleibt. Nach einer Phasentrennung kann das Kohlenmonoxid und Methan enthaltende Kondensat dann durch Rektifikation in Kohlenmonoxid und Methan aufgetrennt werden.

Als günstig erweist es sich, wenn das abgekühlte Kopfprodukt der Abtriebssäule gemeinsam mit der aus dem Sumpf der Methanwäsche abgezogenen Fraktion nach deren Entspannung der Phasentrennung unterzogen wird. Da bei der Entspannung des Sumpfprodukts der Methanwäsche ein Flashgas anfällt, das den überwiegenden Teil des im Sumpfprodukt gelösten Wasserstoffs enthält, bildet sich hierbei ein Zweiphasengemisch, das in seiner Zusammensetzung ungefähr dem abgekühlten Kopfprodukt der Abtriebssäule entspricht.

Deshalb können diese Fraktionen auch gemeinsam weiterverarbeitet werden. Dabei wird zunächst das bei der Entspannung des Sumpfprodukts der Methanwäsche gebildete Flashgas sowie das nichtkondensierte Kopfprodukt der Abtriebssäule abgetrennt und als wasserstofffreiches, Kohlenmonoxid und Methan enthaltendes Restgas abgezogen, das beispielsweise als Heizgas verwendet werden kann. Die verbleibende flüssige Fraktion, die neben Methan und Kohlenmonoxid nur noch geringe Beimengen an Wasserstoff enthält, kann danach in einer Rektifiziersäule in eine Methanfraktion und eine Kohlenmonoxidfraktion zerlegt werden, wobei ein Teil der Methanfraktion für die Methanwäsche herangezogen wird, während der Rest als weiteres Verfahrensprodukt abgezogen und nach Erwärmung beispielsweise ebenfalls als SNG in eine Pipeline eingespeist werden kann.

Selbstverständlich ist es auch möglich, das erfindungsgemässe Verfahren in Gegenwart weiterer inerter Gaskomponenten, beispielsweise Stickstoff, Argon, Helium, durchzuführen. Diese Komponenten werden dann entsprechend ihrem Lösungs- oder Kondensationsverhalten in den einzelnen Fraktionen auftreten, ohne den erfindungsgemässen Verfahrensablauf wesentlich zu verändern.

Nachfolgend werden weitere Einzelheiten des erfindungsgemässen Verfahrens anhand zweier Ausführungsbeispiele erläutert, die in den Figuren schematisch dargestellt sind.

Es zeigen:

Fig. 1 eine Ausführung des erfindungsgemässen Verfahrens mit einer einstufigen partiellen Kondensation auf mittlerem Temperaturniveau und

Fig. 2 eine andere Ausführung des erfindungsgemässen Verfahrens mit einer zweistufigen partiellen Kondensation auf mittlerem Temperaturniveau.

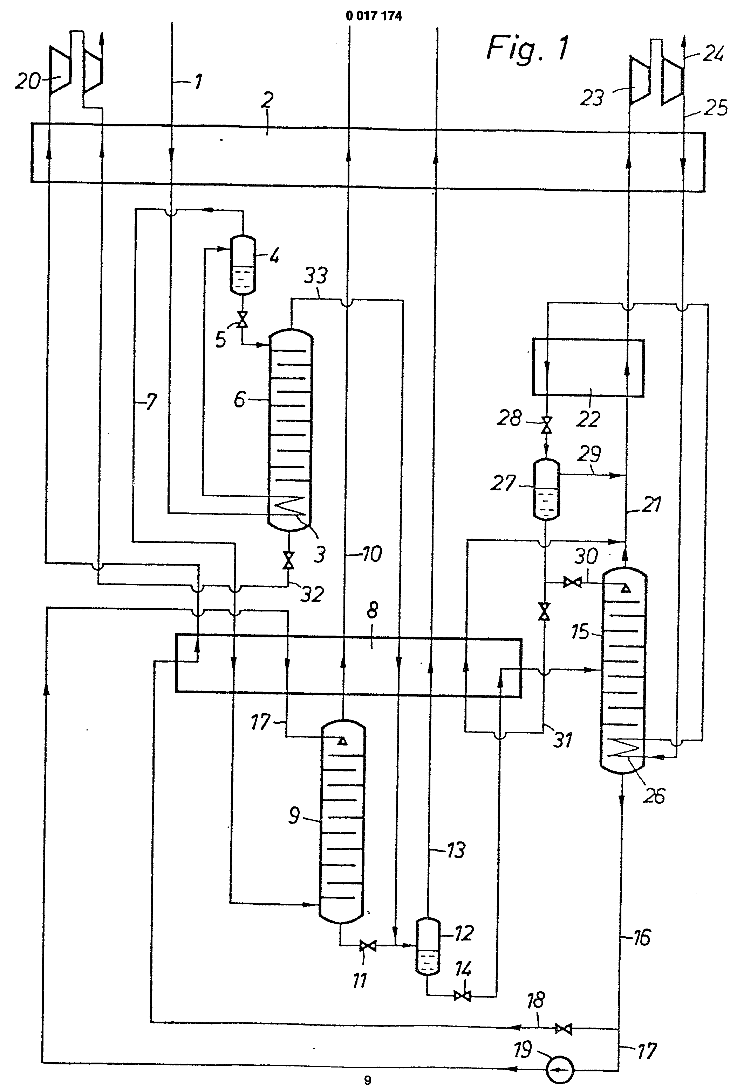

Bei dem Verfahren gemäss Fig. 1 wird Rohgas unter hohem Druck über Leitung 1 einem Wärmetauscher 2 zugeführt, in dem es gegen Zerlegungsprodukte abgekühlt wird. Aus dem Rohgas das zu 44 Vol.-% aus Wasserstoff, 7 Vol.-% aus Kohlenmonoxid und 49 Vol.-% aus Methan besteht, kondensiert dabei bereits ein Grossteil des Methans aus. Nachdem das abgekühlte Gasgemisch die Heizschlange 3 im Sumpf der Abtriebssäule 6 durchströmt hat, gelangt es in den Abscheider 4. Die hier abgetrennte flüssige Fraktion wird im Entspannungsventil 5 auf einen mittleren Druck entspannt und anschliessend in den oberen Bereich der Abtriebsäule 6 eingespeist. Die im Abscheider 4 gasförmig anfallende Fraktion wird über Leitung 7 dem Wärmetauscher 8 zugeführt, in dem eine Abkühlung auf die tiefe Temperatur der Methanwäsche erfolgt. Im Wärmetauscher 8 kondensiert das im Rohgas enthaltene Methan sowie Teile des Kohlenmonoxids. Dieses abgekühlte Gas wird in den unteren Bereich einer Waschsäule 9 eingespeist, in der mittels unterkühltem reinen Methan das Kohlenmonoxid aus der Gasphase ausgewaschen wird. Im Kopf der Waschsäule 9 fällt somit eine gereinigte Wasserstofffraktion ab, die über Leitung 10 abgezogen, in den Wärmetauschern 8 und 2 angewärmt und dann als Produktstrom abgegeben wird. Im Sumpf der Waschsäule 9 fällt ein Gemisch an, das das gesamte gelöste Kohlenmonoxid, das in der Gasfraktion des Abscheiders 4 noch enthaltene Methan, das als Waschmittel verwendete Methan sowie einen kleinen Anteil gelösten Wasserstoffs enthält. Das Sumpfprodukt wird abgezogen und im Entspannungsventil 11 auf einen mittleren Druck entspannt, wobei der gelöste Wasserstoff praktisch vollständig ausgast und im nachfolgenden Abscheider 12 abgetrennt wird. Dieses wasserstoffreiche Flashgas wird über Leitung 13 abgezogen, in den Wärmetauschern 8 und 2 angewärmt und abgegeben; es kann beispielsweise als Heizgas verwendet werden. Die im Abscheider 12 verbliebene flüssige Fraktion wird im Entspannungsventil 14 weiter entspannt, anschliessend im Wärmetauscher 8 teilverdampft und in den mittleren Bereich einer Rektifiziersäule 15 eingespeist. In der Rektifiziersäule 15 wird das Kohlenmonoxid-Methan-Gemisch in eine im Sumpf anfallende reine Methanfraktion sowie eine als Kopfprodukt anfallende reine Kohlenmonoxidfraktion zerlegt. Das im Sumpf gebildete Methan wird über Leitung 16 abgezogen und in zwei Teilströme 17 und 18 aufgeteilt. Über Teilstrom 17 wird das für die Methanwäsche benötigte Waschmittel abgezweigt, mittels der Pumpe 19 auf den Druck der Methanwäsche gebracht und nach Unterkühlung im Wärmetauscher 8 auf den oberen Bereich der Säule 9 als Waschflüssigkeit aufgegeben. Überschüssiges Methan wird über Leitung 18 abgeführt, im Wärmetauscher 8 verdampft, anschliessend im Wärmetauscher 2 angewärmt und als Verfahrensprodukt abgegeben. Da es unter einem niedrigen Druck, im allgemeinen zwischen 1 und 3 bar, anfällt, wird im Kompressor 20 eine Verdichtung auf den Druck eines Erdgasnetzes, d.h. in der Regel auf einen Druck zwischen 30 und 80 bar, vorgenommen.

Das im Kopf der Rektifiziersäule 15 anfallende Kohlenmonoxid wird über Leitung 21 abgezogen, in den Wärmetauschern 22 und 2 angewärmt und gelangt in den Verdichter 23. Produkt-Kohlenmonoxid kann über Leitung 24 bei einer gewünschten Druckstufe abgezogen werden. Der Verdichter 23 wird als Verdichter eines Kältekreislaufes verwendet, um die für die Rektifikation 15 und die Abkühlung des Gases in den Leitungen 7 und 33 erforderliche Kälte zu erzeugen. Dazu wird der über Leitung 25 im Kreislauf geführte Teilstrom des Kohlenmonoxids im Wärmetauscher 2 abgekühlt, anschliessend im Sumpf der Rektifiziersäule 15 in der Heizschlange 26 kondensiert, wobei gleichzeitig der Rektifikationsdampf erzeugt wird, danach im Wärmetauscher 22 gegen Produkt-Kohlenmonoxid unterkühlt und in den Kohlenmonoxid-Sammelbehälter 27 entspannt. Das bei der Entspannung im Ventil 28 gebildete Flashgas gelangt über Leitung 29 direkt in die Produktleitung 21 und wird gemeinsam mit dem Kopfprodukt der Rektifiziersäule 15 wieder angewärmt. Die Flüssigkeit aus dem Kohlenmonoxid-Sammelbehälter 27 dient zum einen Teil als Säulenrücklauf 30, der der Rektifiziersäule 15 im oberen Bereich aufgegeben wird und zum anderen Teil 31 als Kältemittel im Wärmetauscher 8.

Aus dem der Abtriebssäule 6 eingespeisten Kondensat aus dem Abscheider 4 werden die in SNG störenden Verunreinigungen Wasserstoff und Kohlenmonoxid mittels der rohgasbeheizten Heizschlange 3 über Kopf abgetrieben. Das abgetrennte Sumpfprodukt stellt mengenmässig ungefähr die Hälfte des im Rohgas enthaltenen Methans dar. Es wird über Leitung 32 abgezogen, im Wärmetauscher 2 verdampft, angewärmt und anschliessend in eine mittlere Druckstufe des Verdichters 20 abgegeben.

Das Kopfprodukt der Abtriebssäule 6, das neben Wasserstoff und Kohlenmonoxid auch noch viel Methan enthält, wird über Leitung 33 abgezogen und im Wärmetauscher 8 auf die Temperatur der Methanwäsche 9 abgekühlt. Da hierbei praktisch alles Methan sowie ein Teil des Kohlenmonoxids kondensiert, wird das abgekühlte Gemisch anschliessend mit dem im Ventil 11 entspannten Sumpfprodukt der Methanwäsche vereinigt und in den Abscheider 12 geführt.

Wenn, wie im vorliegenden Beispiel angenommen, der Rohgasdruck hoch ist, beispielsweise über 70 bar, und ein hoher Methangehalt im Rohgas vorliegt, ist der bei den Entspannungen 5, 11, 28 des Gasgemisches auftretende Joule-Thomson-Effekt im allgemeinen so stark, dass auf eine besondere Kälteanlage zur Abkühlung des Rohgases auf das mittlere Temperaturniveau verzichtet werden kann. Sofern dieses im speziellen Einzelfall jedoch einmal nicht möglich ist, genügt es, eine zusätzliche Kühlung im Wärmetauscher 2 durch Installation einer üblichen Kälteanlage auf einem relativ hohen Temperaturniveau vorzusehen.

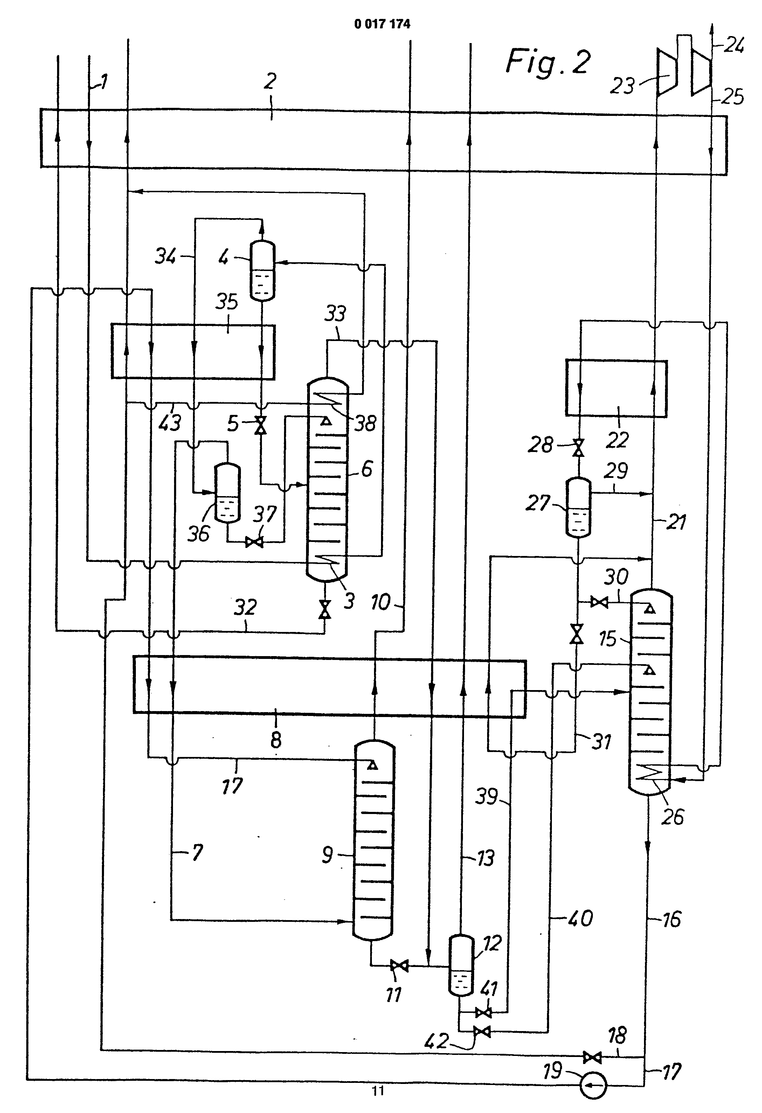

Bei dem in Fig. 2 dargestellten Ausführungsbeispiel wird Rohgas in einer Menge von 100 000 Nm³/h unter einem Druck von 75 bar und bei einer Temperatur von 308 K über Leitung 1 herangeführt. Es enthält 44 Vol.-% Wasserstoff, 7 Vol.-% Kohlenmonoxid und 49 Vol.-% Methan. Nach Abkühlung im Wärmetauscher 2 und in der Sumpfheizung 3 der Abtriebssäule 6 fallen im Abscheider 4 42 000 Nm³/h Kondensat sowie 58 000 Nm³/h einer gasförmigen Fraktion an. Das Kondensat besteht zu 84% aus Methan und enthält daneben noch 9% Wasserstoff und 7% Kohlenmonoxid. Die gasförmige Fraktion besteht zu 69% aus Wasserstoff und weist ausserdem einen Methangehalt von 24% sowie 7% Kohlenmonoxid auf. Die Temperatur beider Fraktionen liegt bei 147 K. Die gasförmige Fraktion aus dem Abscheider 4 wird über Leitung 34 in einem Wärmetauscher 35 weiter auf 128 K abgekühlt, wobei weitere 11 000 Nm³/h des Gasgemisches kondensieren. Im Abscheider 36 wird deshalb eine zweite Phasentrennung vorgenommen, um das Kondensat, das zu 82% aus Methan besteht und jeweils 9% Wasserstoff und Kohlenmonoxid enthält, von der zu 83% aus Wasserstoff bestehenden und 6% Kohlenmonoxid sowie 11% Methan enthaltenden gasförmigen Fraktionen abzutrennen.

Die Kondensate aus den Abscheidern 4 und 36 werden der Abtriebssäule 6 entsprechend den jeweiligen Gleichgewichtsbedingen getrennt aufgegeben. Das Kondensat aus Abscheider 4 wird zunächst im Wärmetauscher 35 unterkühlt und dann nach Entspannung im Drosselventil 5 in den mittleren Bereich der Säule 6 aufgegeben, während das Kondensat aus Abscheider 36 nach Entspannung 37 auf den Druck der Abtriebssäule 6 in den oberen Bereich dieser Säule eingeführt wird.

Die Abtriebssäule 6 wird bei einem Druck von 7,5 bar betrieben und arbeitet bei Temperaturen zwischen 144 K im Sumpf und 128 K am Kopf der Säule. In diesem Ausführungsbeispiel ist die Abtriebssäule 6 zusätzlich mit einem Kopfkühler 38 versehen, um eine Rückkondensation eines Teiles des im Kopfprodukt enthaltenen Methans und damit eine Erhöhung der Methanausbeute in dieser Verfahrensstufe zu erreichen.

Aus dem Sumpf der Abtriebssäule werden stündlich 33 000 Nm³/h praktisch reinen Methans abgezogen, das über Leitung 32 dem Wärmetauscher 2 zugeführt wird und dann als Mitteldruck-Methan vorliegt. Es kann beispielsweise wieder, wie in der Fig. 1 angegeben, verdichtet und einer Erdgasleitung zugeführt werden. Als Kopfprodukt der Abtriebssäule 6 werden über Leitung 33 20 000 Nm³/h einer gasförmigen Fraktion abgezogen, die zu 55% aus Methan, 25% Wasserstoff und 20% Kohlenmonoxid besteht.

Die weitere Verfahrensführung entspricht in diesem Beispiel im wesentlichen dem bereits in Fig. 1 beschriebenen Verfahren. Unterschiede bestehen lediglich in der Beschickung der Rektifiziersäule 15, die in diesem Fall dadurch erfolgt, dass das Kondensat aus dem Abscheider 12 in zwei Teilströme 39 und 40 aufgeteilt wird, die nach jeweiliger Entspannung 41, 42 auf den Druck der Rektifiziersäule getrennt geführt werden. Während der Teilstrom 39, entsprechend dem Verfahren der Fig. 1, nach Teilverdampfung in einen mittleren Bereich der Säule 15 eingespeist wird, gelangt der Teilstrom 40 direkt als Rücklauf in einen höher gelegenen Bereich der Säule 15. Ein weiterer Unterschied besteht in der Führung der Methanfraktion 16, die in der Rektifiziersäule 15 gewonnen wird. Der für die Wäsche verwendete Teilstrom 17 wird in diesem Fall zunächst im Wärmetauscher 35 und dann im Wärmetauscher 8 unterkühlt, bevor er der Waschsäule als Rücklauf aufgegeben wird. Der andere Teilstrom 18 wird nicht mehr durch den Wärmetauscher 8 geführt, sondern in den Wärmetauschern 35 und 2 verdampft und angewärmt. Vor der Anwärmung wird von diesem Teilstrom 18 ein weiterer Teilstrom 43 abgezweigt, der im Kopfkühler 38 der Abtriebssäule 6 verdampft und anschliessend wieder in den Teilstrom 18 zurückgeführt wird, bevor die Erwärmung auf Umgebungstemperatur im Wärmetauscher 2 erfolgt. Das Niederdruck-Methan kann dann, entsprechend dem Beispiel der Fig. 1, beispielsweise ebenfalls verdichtet und in ein Erdgasnetz abgegeben werden.

Für den auf die Vorabscheidung des Methans folgenden Verfahrensablauf seien nachfolgend noch die charakteristischen Daten angegeben.

In der Methanwäsche 9 wird das über Leitung 7 eingespeiste Gasgemisch mit 11 000 Nm³/h reinen Methans einer Temperatur von 92,5 K gewaschen. Über Leitung 10 wird auf diese Weise kohlenmonoxidfreier Wasserstoff in einer Menge von 39 000 Nm³/h gewonnen, der lediglich 2% Methan als Verunreinigung enthält. Im Sumpf der Waschsäule fallen 19 000 Nm³/h einer Flüssigkeit an, die zu 79% aus Methan besteht und 16% Kohlenmonoxid sowie 5% Wasserstoff enthält. Die Sumpftemperatur liegt bei 95,5 K.

Nach Entspannung des Sumpfproduktes auf 7,3 bar und Vermischung mit dem Kopfprodukt der Abtriebssäule 6 fällt im Abscheider 12 ein Flashgas in einer Menge von 6600 Nm³/h an, das 83% Wasserstoff, 14% Kohlenmonoxid und 3% Methan enthält, während die verbleibende flüssige Fraktion (32 400 Nm³/h) zu 80% aus Methan besteht, 19% Kohlenmonoxid und 1% Wasserstoff enthält. Im Sumpf der Rektifiziersäule 15 fallen 25 800 Nm³/h Methan an, von denen nach Abtrennung des Waschmethans 14 800 Nm³/h als Verfahrensprodukt bei einer Temperatur von 126 K und einem Druck von 2,5 bar über Leitung 18 abgezogen werden.

Ein Vergleich mit der über Leitung 32 gewonnenen Methanmenge zeigt, dass 69% des abgetrennten Methans in der Vorabscheidung auf mittlerem Temperaturniveau gewonnen wurden und dass lediglich 31% bei tiefer Temperatur unter erhöhtem Aufwand abgetrennt werden müssen.

Schliesslich fällt als Kopfprodukt der Rektifiziersäule noch eine Kohlenmonoxidfraktion in einer Menge von 6600 Nm³/h an, die zu 92% aus Kohlenmonoxid besteht und daneben 8% Wasserstoff enthält.

Die vorstehend genannten Prozentangaben beziehen sich jeweils auf Volumenprozente.

**Patentansprüche**

1. Verfahren zum Zerlegen eines im wesentlichen Wasserstoff, Methan und Kohlenmonoxid enthaltenden Gasgemisches mit einer bei tiefer Temperatur erfolgenden Methanwäsche, die eine Waschsäule (9) und eine Rektifiziersäule (15) enthält, wobei das Gasgemisch zunächst bei einer über der Temperatur der Methanwäsche liegenden Temperatur einer partiellen Kondensation unterzogen und die dabei gasförmig verbleibende Fraktion der Methanwäsche zugeführt wird, dadurch gekennzeichnet, dass die bei der partiellen Kondensation kondensierte Fraktion in eine bei höherer Temperatur als die Rektifiziersäule betriebene Abtriebssäule (6), in der Methan als Sumpfprodukt und ein an Wasserstoff und Kohlenmonoxid angereichertes Kopfprodukt gewonnen wird, geführt wird.

2. Verfahren nach Anspruch 1, dadurch gekennzeichnet, dass der Kopf der Abtriebssäule durch mindestens einen Teilstrom des Sumpfprodukts der Rektifiziersäule gekühlt wird.

3. Verfahren nach Anspruch 1 oder 2, dadurch gekennzeichnet, dass die partielle Kondensation im Temperaturbereich zwischen 180 K und etwa 128 K durchgeführt wird.

4. Verfahren nach einem der Ansprüche 1 bis 3, dadurch gekennzeichnet, dass die Abtriebssäule bei einem geringeren Druck als die Waschsäule betrieben wird.

5. Verfahren nach einem der Ansprüche 1 bis 4, dadurch gekennzeichnet, dass die partielle Kondensation mehrstufig durchgeführt wird.

6. Verfahren nach Anspruch 5, dadurch gekennzeichnet, dass die kondensierten Fraktionen einer gemeinsamen Abtriebssäule zugeführt werden.

7. Verfahren nach einem der Ansprüche 1 bis 6, dadurch gekennzeichnet, dass das Kopfprodukt der Abtriebssäule auf die tiefe Temperatur abgekühlt und dabei anfallendes Kondensat in einer Phasentrennung abgeschieden wird.

8. Verfahren nach Anspruch 7, dadurch gekennzeichnet, dass die aus der Waschsäule abgezogene, im wesentlichen Methan und Kohlenmonoxid enthaltende flüssige Fraktion entspannt und gemeinsam mit dem abgekühlten Kopfprodukt der Abtriebssäule einer Phasentrennung unterzogen wird.

9. Verfahren nach Anspruch 7 oder 8, dadurch gekennzeichnet, dass die bei der Phasentrennung anfallende flüssige Fraktion in der Rektifiziersäule mittels Rektifikation in eine Methanfraktion und eine Kohlenmonoxidfraktion zerlegt wird.

**Revendications**

1. Procédé de séparation des constituants d'un mélange gazeux contenant essentiellement de l'hydrogène, du méthane et du monoxyde de carbone par lavage à l'aide de méthane à basse température dans une zone de lavage comprenant une colonne de lavage et une colonne de régénération, le mélange gazeux étant tout d'abord soumis à une température supérieure à celle du lavage au méthane, à une condensation partielle et la fraction gazeuse restante étant envoyée vers la zone de lavage, caractérisé en ce que la fraction condensée pendant la condensation partielle est dirigée vers une colonne de fractionnement fonctionnant à une température supérieure à celle de la colonne de régénération, la colonne de fractionnement produisant du méthane comme produit de pied et un produit de tête enrichi en hydrogène et en monoxyde de carbone.

2. Procédé suivant la revendication 1, caractérisé en ce que la tête de la colonne de fractionnement est refroidie par au moins un courant partiel du produit de cuve de la colonne de régénération.

3. Procédé suivant la revendication 1 ou 2, caractérisé en ce que la condensation partielle est effectuée dans une plage de température comprise entre 180 °K et environ 128 °K.

4. Procédé suivant une quelconque des revendications 1 à 3, caractérisé en ce que la colonne de fractionnement fonctionne sous une pression inférieure à celle de la colonne de lavage.

5. Procédé suivant une quelconque des revendications 1 à 4, caractérisé en ce que la condensation partielle est exécutée en plusieurs étages.

6. Procédé suivant la revendication 5, caractérisé en ce que les fractions condensées sont dirigées vers une colonne de fractionnement commune.

7. Procédé suivant une quelconque des revendications 1 à 6, caractérisé en ce que le produit de tête de la colonne de fractionnement est refroidi à ladite basse température et que le condensat correspondant est séparé par une séparation de phases.

8. Procédé suivant la revendication 7, caractérisé en ce que la fraction liquide provenant de la colonne de lavage et contenant surtout du méthane et du monoxyde de carbone est détendue et soumise, avec le produit de tête refroidi de la colonne de fractionnement, à une séparation de phases.

9. Procédé suivant la revendication 7 ou 8, caractérisé en ce que la fraction liquide obtenue pendant la séparation de phases est séparée dans la colonne de régénération par rectification en une fraction méthane et une fraction monoxyde de carbone.

**Claims**

1. A process for the separation of a gas mixture, the basic constituents of which are hydrogen, methane and carbon monoxide, comprising a methane wash which is carried out at a low temperature and which includes a washing column and a regeneration column, in which the gas mixture is first subjected to partial condensation at a temperature above the temperature of the methane wash, and the fraction which remains in gaseous form is fed to the methane wash, characterised in that the fraction which is condensed during the partial condensation is fed to a separating column which operates at a higher temperature than the regenerating column and in which there are obtained methane as sump product and a head product enriched with hydrogen and carbon monoxide.

2. A process according to Claim 1, characterised in that the head of the separating column is cooled by at least one sub-stream of the sump product of the regenerating column.

3. A process according to Claim 1 or Claim 2, characterised in that the partial condensation is carried out in the temperature range of between 180 K and about 128 K.

4. A process according to one of Claims 1 to 3, characterised in that the separating column operates at a lower pressure than does the washing column.

5. A process according to one of Claims 1 to 4, characterised in that the partial condensation is carried out in a plurality of steps.

6. A process according to Claim 5, characterised in that the condensed fractions are fed to a common separating column.

7. A process according to one of Claims 1 to 6, characterised in that the head product from the separating column is cooled to the low temperature and condensate thereby formed is precipitated in a phase separation step.

8. A process according to Claim 7, characterised in that the liquid fraction which is withdrawn from the washing column and which basically contains methane and carbon monoxide, is expanded and subjected to phase separation, together with the cooled head product from the separating column.

9. A process according to Claim 7 or Claim 8, characterised in that the liquid fraction which forms during the phase separation is separated by rectification in the regenerating column into a methane fraction and a carbon monoxide fraction.

Fig. 1

0 017 174

Fig.2